# EUROPEAN PATENT SPECIFICATION

(11) **EP 0 835 866 B1**
(45) Date of publication and mention of the grant of the patent: **16.08.2001**
(21) Application number: 97307982.5
(22) Date of filing: 09.10.1997
(51) Int. Cl.: C07D 275/04

(54) **Process for preparation of 3-piperazinylbenzisothiazoles**
Verfahren zur Herstellung von 3-Piperazinylisothiazolen
Procédé de préparation de 3-pipérazinyl-isothiazoles

(30) Priority: 11.10.1996 JP 26988896
(43) Date of publication of application: 15.04.1998
(73) Proprietor: SUMITOMO SEIKA CHEMICALS CO., LTD., Kako-gun Hyogo-ken (JP)
(72) Inventor: Goda, Hiroshi, Sumitomo Seika Chem. Co. Ltd. Resea, Kako-gun, Hyogo-ken (JP); Sakamoto, Junichi, Sumitomo Seika Chem. Co. Ltd. R, Kako-gun, Hyogo-ken (JP); Sakaue, Shigeki, Sumitomo Seika Chem. Co. Ltd. Res, Kako-gun, Hyogo-ken (JP); Kajihara, Sakae, Sumitomo Seika Chem. Co. Ltd. Res, Kako-gun, Hyogo-ken (JP); Todo, Miki, Sumitomo Seika Chem. Co. Ltd. Research, Kako-gun, Hyogo-ken (JP)
(74) Representative: Coleiro, Raymond

(56) References cited:
- EP-A- 0 741 129
- GB-A- 2 163 432

## Description

The present invention relates to a process for preparing 3-piperazinylbenzisothiazoles which are useful compounds as intermediates for production of pharmaceutical preparations.

For preparing 3-piperazinylbenzisothiazole derivatives, two known processes have been mainly used. One of them is a process in which 3-halo-1,2-benzisothiazole is reacted with a piperazine compound according to the following reaction scheme (1) : 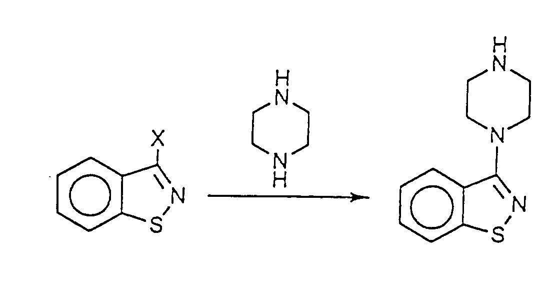 (JP-A-63-083067; JP-A-63-083085; EP-A-0196096; J.Chem. Soc., Perkin. Trans., (1988), 1(8), 2141; DE-A-3530089; J.Med. Chem., (1986), 29(3), 359), and the other is a process which is disclosed in the applicants' own earlier JP-A-08-291134, which can be represented by the reaction scheme (2): 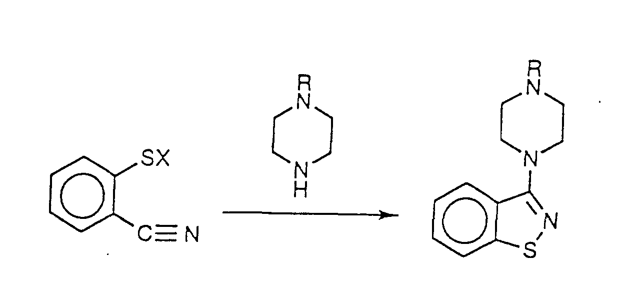 wherein X represents Cl or Br, and R represents H, an alkyl group having 1 to 6 carbon atoms or a substituted alkylene group having 1 to 6 carbon atoms.

However, the above known techniques require a largely excessive amount if piperazine and a very long reaction time and lead to low yield. Thus, they are not regarded as industrially effective processes.

In view of this, it was difficult, in an industrially effective manner, to prepare 3-piperazinylbenzisothiazoles by any known process.

The present invention addresses the problem of providing an industrially effective process for preparing 3-piperazinylbenzisothiazoles.

Thus, in view of the above circumstances, we conducted intensive studies in order to provide an easy process for economically effectively preparing 3-piperazinylbenzisothiazoles and thus solve the above problem. As a result, we found surprisingly that, in a reaction for preparing 3-piperazinylbenzisothiazoles by reacting 3-halo-1,2-benzisothiazole or 2-cyanobenzenesulfenyl halide with a piperazine compound, when the reaction is performed in the presence of an alkylene glycol derivative, advantages such as a reduction in the amount of piperazine to be used, improvement in yield and reduction in reaction time can be attained.

The mechanism of this reaction is not clear but such advantages may result because the alkylene glycol derivative manifests a phase transfer catalyst-like activity and enhances the solubility of the piperazine compound in the reaction system.

Thus, in accordance with the present invention there is provided a process, which can be carried out economically and effectively for preparing a 3-piperazinylbenzisothiazole represented by the general formula (IV): 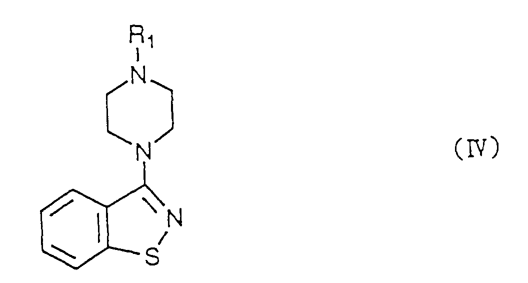 wherein R₁ represents a hydrogen atom or an alkyl group having 1-6 carbon atoms, which process comprises:
reacting a 3-halo-1,2-benzisothiazole represented by the general formula (I): 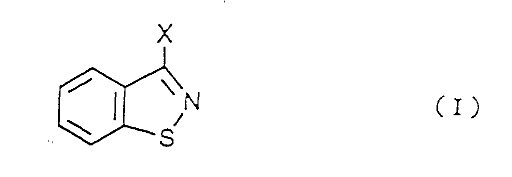 wherein X represents Cl or Br, or a 2-cyanobenzenesulfenyl halide represented by the general formula (V): 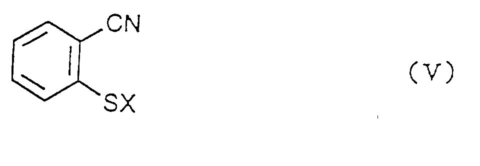 wherein X is as defined above, with a piperazine compound represented by the general formula (II): 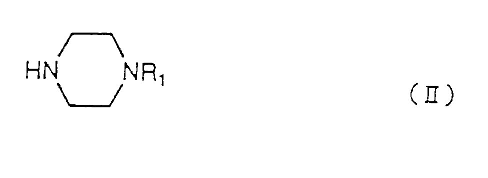 wherein R₁ is as defined above, in the presence of an alkylene glycol derivative represented by the general formula (III) : 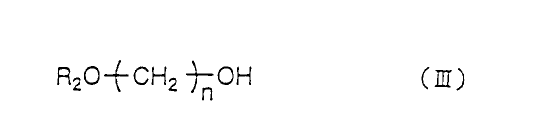 wherein R₂ represents a hydrogen atom or a linear or branched alkyl group having 1 to 4 carbon atoms and n represents 2-4.

Embodiments of the present invention will be explained in detail below.

The 3-halo-1,2-benzisothiazole used in the present invention is represented by the general formula (I) and examples thereof are 3-chloro-1,2-benzisothiazole and 3-bromo-1,2-benzisothiazole.

The 2-cyanobenzenesulfenyl halide compound which can be used in the present invention as an alternative to the above 3-halo-1,2-benzisothiazole is represented by the general formula (V) and can be easily obtained by halogenating a 2-cyanophenylthio derivative according to a process described in the applicants' own earlier JP-A-08-291134, and examples thereof are 2-cyanobenzenesulfenyl chloride and 2-cyanobenzenesulfenyl bromide.

The piperazine compound used in the present invention is represented by the general formula (II) and examples thereof are piperazine, 1-alkyl-piperazines such as 1-methyl-piperazine, 1-ethyl-piperazine and 1-n-butyl-piperazine. In particular, piperazine is preferably used.

The amount of the piperazine compound to be used is preferably in a range of 1 to 10 moles, more preferably 2 to 4 moles relative to 1 mole of 3-halo-1,2-benzisothiazole represented by the general formula (I) or 2-cyanobenzenesulfenyl halide represented by the general formula (V).

Furthermore, the alkylene glycol derivative used in the present invention is represented by the general formula (III) and examples thereof are ethylene glycol, propylene glycol, ethylene glycol monomethyl ether and ethylene glycol monoethyl ether. In particular, ethylene glycol is preferably used.

The amount of the alkylene glycol derivative to be used is preferably in a range of 0.1 to 10 parts by weight, more preferably 0.5 to 5 parts by weight relative to 1 part by weight of 3-halo-1,2-benzisothiazole represented by the general formula (I) or 2-cyanobenzenesulfenyl halide represented by the general formula (V).

When the amount of the alkylene glycol derivative to be used is below this range, its effect may not be observed and, when the amount exceeds this range, then again its effect may no longer be observed, conversely leading to disadvantages in carrying out the respective reaction operations.

3-Piperazinylbenzisothiazoles which are an end product of a process embodying the present invention are represented by the general formula (IV) and example thereof are 3-(1-piperazinyl)-1,2-benzisothiazole, 3-(4-ethyl-1-piperazinyl)-1,2-benzisothiazole, 3-(4-n-butyl-1-piperazinyl)-1,2-benzisothiazole and 3-(4-cyclohexyl-1-piperazinyl)-1,2-benzisothiazole.
Alternatively, these compounds may be isolated as a salt of a mineral acid such as the hydrochloride or sulfate under acidic conditions in the presence of, for example, hydrochloric acid or sulfuric acid.

A reaction temperature in the process for preparing 3-piperazinylbenzisothiazoles as described is normally in the range of about 70 to about 150°C, preferably about 100 to about 130°C. When the reaction temperature is lower than 70°C, the reaction rate may become slow. On the other hand, when the reaction temperature is higher than 150°C, side reactions may occur, causing a reduction in yield.

A solvent is not necessarily required for the reaction and a non-solvent reaction is preferably used although the reaction may be performed in a solvent. In this case, examples of the solvent to be used are hydrocarbons such as cyclohexane and heptane, aromatic hydrocarbons such as benzene, toluene, xylene, chlorobenzene, dichlorobenzene and trichlorobenzene and polar solvents such as N,N-dimethylformamide and dimethyl sulfoxide. Normally, the amount to be used is, but not limited to, 0.1 to 10 parts by weight relative to 1 part by weight of the 3-halo-1,2-benzisothiazole represented by the general formula (I) or 2-cyanobenzenesulfenyl halide represented by the general formula (V).

The thus obtained 3-piperazinylbenzisothiazole represented by the general formula (IV) may be easily isolated and purified from the reaction mixture according to a conventional method such as crystallization.

Surprisingly, by using the process of the present invention, 3-piperazinylbenzisothiazoles which are an end product can be obtained in about 1/2 to 1/12 the reaction time, with about 1/2 to 1/6 of the amount of the piperazine compound, and in a yield higher by about 20% to 50% as compared with previous known processes.

The following Examples illustrate in more detail embodiments of the present invention.

### Example 1

215.4 g (2.50 mol) of piperazine, 50.0 g of chlorobenzene and 100.0 g of ethylene glycol were placed in a four-neck 1000 ml flask equipped with a stirrer, a thermometer, a dropping funnel and a condenser and 169.5 g (1.00 mol) of 3-chloro-1,2-benzisothiazole was added dropwise in the molten state at about 120°C over 1 hour while stirring, after which stirring was continued for 2 hours to complete the reaction. After removing excess piperazine with water, the reaction mixture was made acidic with hydrochloric acid and extracted into an aqueous layer, which was made alkaline with aqueous sodium hydroxide to obtain 202.4 g (m.p. 89-90°C) of 3-(1-piperazinyl)-1,2-benzisothiazole as crystals. Yield from 3-chloro-1,2-benzisothiazole was 92.4%.

### Example 2

215.4 g (2.50 mol) of piperazine, 50.0 g of chlorobenzene and 100.0 g of ethylene glycol were placed in a four-neck 1000 ml flask equipped with a stirrer, a thermometer, a dropping funnel and a condenser and 214.1 g (1.00 mol) of 2-cyanobenzenesulfenyl chloride was added dropwise in the molten state at about 120°C over 1 hour while stirring, after which stirring was continued for 2 hours to complete the reaction. After removing excess piperazine with water, the reaction mixture was made acidic with hydrochloric acid and extracted into an aqueous layer, which was made alkaline with aqueous sodium hydroxide to obtain 199.5 g (m.p. 89-90°C) of 3-(1-piperazinyl)-1,2-benzisothiazole as crystals. Yield from 2-cyanobenzenesulfenyl chloride was 91.1%.

### Comparative Example 1

304.2 g (3.53 mol) of piperazine, 7.5 g of chlorobenzene and 39.8 g (0.235 mol) of 3-chloro-1,2-benzisothiazole were placed in a four-neck 1000 ml flask equipped with a stirrer, a thermometer, a dropping funnel and a condenser and the mixture was heated with stirring at about 120°C for 20 hours. After removing excess piperazine with water, the reaction mixture was made acidic with hydrochloric acid and extracted into an aqueous layer, which was made alkaline with aqueous sodium hydroxide to obtain 24.4 g (m.p. 89 to 90°C) of 3-(1-piperazinyl)-1,2-benzisothiazole as crystals. Yield from 3-chloro-1,2-benzisothiazole was 47.4%.

### Comparative Example 2

86.2 g (1.00 mol) of piperazine and 7.5 g of chlorobenzene were placed in a four-neck 500 ml flask equipped with a stirrer, a thermometer, a dropping funnel and a condenser and 53.5 g (0.25 mol) of 2-cyanobenzenesulfenyl chloride was added dropwise in the molten state at about 130°C over 1 hour while stirring, after which stirring was continued for 4 hours to complete the reaction. After removing excess piperazine with water, the reaction mixture was made acidic with hydrochloric acid and extracted into an aqueous layer, aqueous layer, which was made alkaline with aqueous sodium hydroxide to obtain 40.9 g (m.p. 89 to 90°C) of 3-(1-piperazinyl)-1,2-benzisothiazole as crystals. Yield from 2-cyanobenzenesulfenyl chloride was 74.7%.

## Claims

1. A process for preparing a 3-piperazinylbenzisothiazole represented by the general formula (IV): 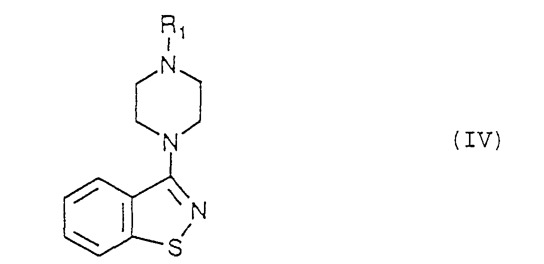 wherein R₁ represents a hydrogen atom or an alkyl group having 1 to 6 carbon atoms, which process comprises:
reacting either
(a) a 3-halo-1,2-benzisothiazole represented by the general formula (I): 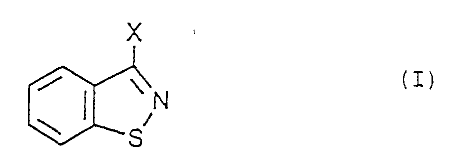 wherein X represents Cl or Br; or
(b) a 2-cyanobenzenesulfenyl halide represented by the general formula (V): 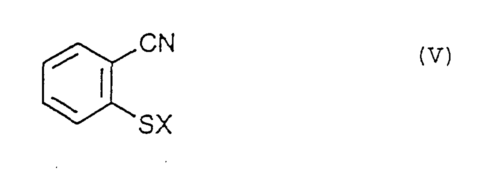
wherein X is as defined above;
with a piperazine compound represented by the general formula (II): 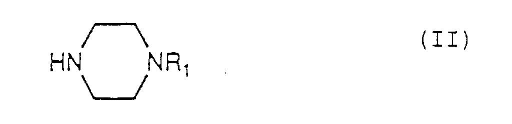 wherein R₁ is as defined above, in the presence of an alkylene glycol derivative represented by the general formula (III):
R₂O(̵CH₂)̵ₙOH (III)
wherein R₂ represents a hydrogen atom or a linear or branched alkyl group having 1 to 4 carbon atoms, and n represents 2 to 4.

2. A process according to claim 1, wherein the piperazine compound represented by the general formula (II) is piperazine.

3. A process according to claim 1 or claim 2, wherein the 3-piperazinylbenzisothiazole represented by the general formula (IV) is 3-(1-piperazinyl)-1,2-benzisothiazole.

4. A process according to any one of claims 1 to 3 wherein the alkylene glycol derivative represented by the general formula (III) is selected from ethylene glycol, propylene glycol, ethylene glycol monomethyl ether and ethylene glycol monoethyl ether.

5. A process according to claim 4, wherein the alkylene glycol derivative represented by the general formula (III) is ethylene glycol.

## Patentansprüche

1. Verfahren zur Herstellung eines 3-Piperazinylbenzisothiazols mit der allgemeinen Formel (IV): 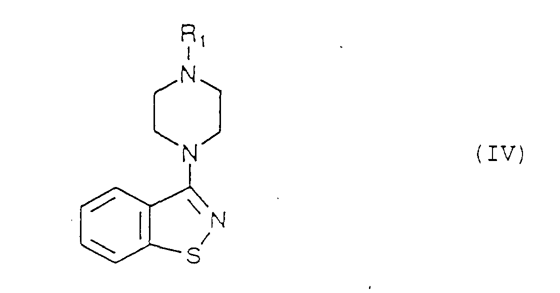 worin R₁ ein Wasserstoffatom oder eine Alkyl-Gruppe mit 1 bis 6 Kohlenstoffatomen ist, umfassend:
Reaktion von
(a) einem 3-Halo-1,2-benzisothiazol mit der allgemeinen Formel (I): 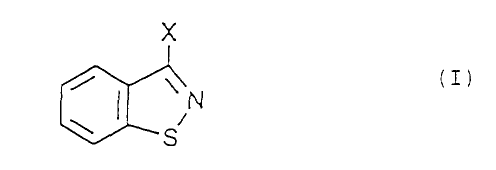 worin X Cl oder Br ist; oder
(b) einem 2-Cyanobenzolsulfenylhalogenid mit der allgemeinen Formel (V): 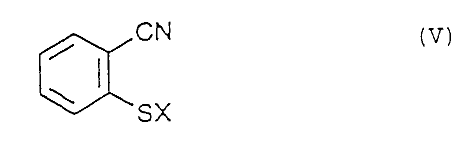 worin X wie oben definiert ist; mit einer Piperazin-Verbindung mit der allgemeinen Formel (II): 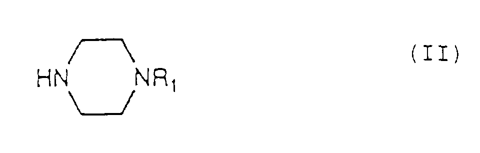 worin R₁ wie oben definiert ist, in der Gegenwart eines Alkylenglycol-Derivates mit der allgemeinen Formel (III): 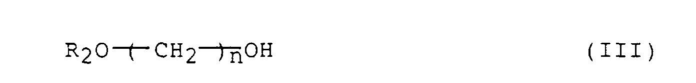
worin R₂ ein Wasserstoffatom oder eine lineare oder verzweigte Alkyl-Gruppe mit 1 bis 4 Kohlenstoffatomen und n 2 oder 4 sind.

2. Verfahren nach Anspruch 1, worin die Piperazin-Verbindung mit der allgemeinen Formel (II) Piperazin ist.

3. Verfahren nach Anspruch 1 oder 2, worin das 3-Piperazinylbenzisothiazol mit der allgemeinen Formel (IV) 3-(1-Piperazinyl)-1,2-benzisothiazol ist.

4. Verfahren nach einem der Ansprüche 1 bis 3, worin das Alkylenglycol-Derivat mit der allgemeinen Formel (III) ausgewählt wird aus Ethylenglycol, Propylenglycol, Ethylenglycolmonomethylether und Ethylenglycolmonoethylether.

5. Verfahren nach Anspruch, worin das Alkylenglycol-Derivat mit der allgemeinen Formel (III) Ethylenglycol ist.

## Revendications

1. Procédé de préparation d'un 3-pipérazinylbenzisothiazole représenté par la formule générale (IV) : 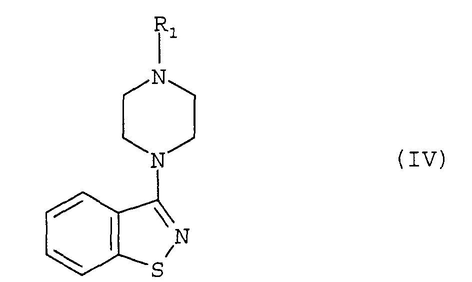 dans laquelle R₁ représente un atome d'hydrogène ou un groupe alkyle ayant de 1 à 6 atomes de carbone, lequel procédé comprend les étapes consistant à :
faire réagir soit
(a) un 3-halo-1,2-benzisothiazole représenté par la formule générale (I) : 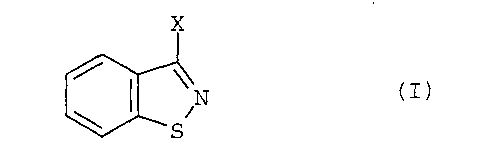 dans laquelle X représente Cl ou Br ; soit
(b) un halogénure de 2-cyanobenzènesulfényle représenté par la formule générale (V) : 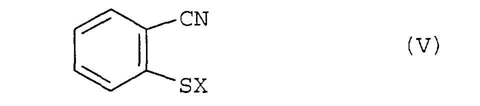 dans laquelle X est tel que défini ci-dessus ;
avec un composé de pipérazine représenté par la formule générale (II) : 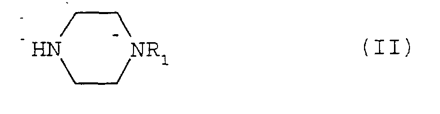 dans laquelle R₁ est tel que défini ci-dessus, en présence d'un dérivé alkylène glycol représenté par la formule générale (III) : 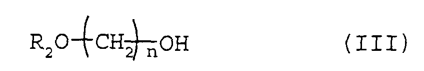 dans laquelle R₂ représente un atome d'hydrogène ou un groupe alkyle linéaire ou ramifié ayant de 1 à 4 atomes de carbone, et n représente 2 à 4.

2. Procédé selon la revendication 1, dans lequel le composé de pipérazine représenté par la formule générale (II) est la pipérazine.

3. Procédé selon la revendication 1 ou la revendication 2, dans lequel le 3-pipérazinylbenzisothiazole représenté par la formule générale (IV) est le 3-(1-pipérazinyl)-1,2-benzisothiazole.

4. Procédé selon l'une quelconque des revendications 1 à 3, dans lequel le dérivé d'alkylène glycol représenté par la formule générale (III) est choisi parmi l'éthylène glycol, le propylène glycol, l'éther monométhylique d'éthylène glycol et l'éther monoéthylique d'éthylène glycol.

5. Procédé selon la revendication 4, dans lequel le dérivé d'alkylène glycol représenté par la formule générale (III) est l'éthylène glycol.
